# Europäisches Patentamt

# European Patent Office

# Office européen des brevets

(11) Publication number: **0 179 025 B1**

# EUROPEAN PATENT SPECIFICATION

(45) Date of publication of patent specification: **29.07.92**

(21) Application number: **85830246.6**

(22) Date of filing: **30.09.85**

(51) Int. Cl.⁵: **C12N 15/57**, C12N 9/56, //C12N15/75

(54) Method for the production of neutral protease.

(30) Priority: **17.10.84 IT 2319084**

(43) Date of publication of application:
**23.04.86 Bulletin 86/17**

(45) Publication of the grant of the patent:
**29.07.92 Bulletin 92/31**

(84) Designated Contracting States:
**AT BE CH DE FR GB LI LU NL SE**

(56) References cited:
**EP-A- 0 077 664**
**EP-A- 0 151 760**

**J. Bacteriol. 154 (1983), 831-837;**

**J. Bacteriol. 160 (1984) 15-21;**

**J. Bacteriol. 159 (1984) 811-819**

**Mol. Gen. Genet. 181 (1981) 434-440**

**J. Bacteriol. 139 (1979) 775-782**

**J. Bacteriol. 119 (1974) 82-91**

Bacillus Genetic Stock Center Catalog of Strains, 2nd edition, page 32

(73) Proprietor: **ENIRICERCHE S.p.A.**

**I-20097 S. Donato Milanese(IT)**

(72) Inventor: **Toma, Salvatore**
**Viale Caterina da Forli, 5**
**I-20146 Milan(IT)**
Inventor: **Del Bue, Marina**
**Viale Gian Galeazzo,7**
**I-20136 Milan(IT)**
Inventor: **Grandi, Guido**
**Nona Strada, 4**
**I-20090 Segrate, Milan(IT)**

(74) Representative: **Vannini, Torquato et al**
**JACOBACCI-CASETTA & PERANI S.p.A. 7 Via Visconti di Modrone**
**I-20122 Milan(IT)**

**Description**

The present invention relates to the field of molecular biology and, in particular, to the construction by genetic engineering techniques, of strains which produce large quantities of proteins.

More particularly, the present invention relates to a method for the production of the exoenzyme neutral protease, comprising the construction of a hybrid recombinant plasmid containing the gene which codes for the neutral protease, the preparation of a mutant strain of Bacillus subtilis, the introduction of the hybrid recombinant plasmid into the mutant strain, the isolation of clones containing the hybrid recombinant plasmid, the growth of the clones in a liquid culture medium under aerobic conditions and, finally, the isolation of the neutral protease from the culture medium.

It is known the use of neutral protease of bacterial origin in industrial fields such as, for example, the food and chemical industries and particularly in the composition of detergents. It is also know how to produce these enzymes by fermentation processes which include the growth, in a suitable culture medium, of microorganisms which naturally contain the gene that codes for the enzyme. Among the microorganisms which produce proteolytic enzymes, those belonging to the Bacillus genus are of particular interest.

These known processes are somewhat onerous due to the number of stages required and, above all, the poor overall production yields.

With the advent of recombinant DNA technology, it is now possible to construct microorganisms which produce large quantities of proteins.

In accordance with U.S. Patent 4237224, a gene which codes for a particular protein is isolated from a donor strain and introduced into a suitable host cell with the use of special DNA molecules called vectors.

The vector molecules used in genetic engineering experiments are able to replicate autonomously in the host cell so that there are generally between 30 and 50 copies present in the end.

It follows that, when an exogenous gene is inserted in the vector and the modified vector, the so-called hybrid recombinant plasmid, is introduced into the host cell, the number of copies of the gene in the cell is equal to that of the vector.

This results in a higher synthesis of the cloned product by the phenomenon known as "gene dosage".

As is known, DNA is transcribed in the cell by the enzyme RNA polymerase into messenger RNA and this, in its turn, is translated into protein by the coordinated action of the ribosomes and a vast series of enzymes and cell products.

Given the high efficiency of the transcription and translation system of the cell if the number of copies of a particular gene is increased, the production of its genetic product is consequently also increased.

J.Bacteriol 160, October 1984, pages 15-21, describes the cloning of the gene which codes for the neutral protease of Bacillus subtilis GSY 264 (BGSC 1A72), which is a strain which does not produce protease in high yield, its production yield being comparable to those obtained from wild type strains such as BG16 and 168.

The above quoted reference also describes the construction of plasmid pNPR10 comprising the structural gene of neutral protease and the region of approximately 900 bP which contains the regulatory signals for expression. This plasmid is then used to transform B.subtilis strain BG84 containing a mutation in the structural gene of subtilisin, in order to produce neutral protease. The data given in table 2 show that BG84 (pNPR10) produces an amount of neutral protease in a ratio of 1:30 with respect to the wild type strain BG16 which is not an overproducing strain of neutral protease. The increase of expression and production of neutral protease is therefore due to the presence of several copies of the plasmid pNPR10 in the transformed strain.

The method according to the present invention allows to obtain an increased yield in the production of B.subtilis neutral protease.

The subject of the present invention is a method for the production of Bacillus subtilis neutral protease comprising:

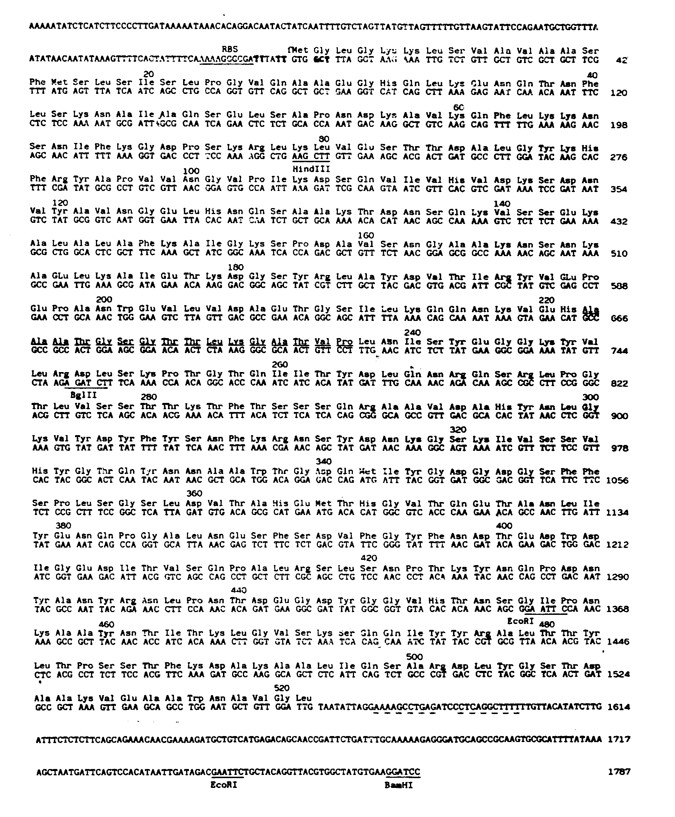

(a) isolating, from the chromosomal DNA of Bacillus subtilis BGSG 1A341, the gene coding the neutral protease having the sequence recited in Figure 3,

(b) constructing a hybrid recombinant plasmid containing the gene obtained in step (a),

(c) introducing the hybrid recombinant plasmid of step (b) into Bacillus subtilis SMS 108 (NRRL-B-1589) having a mutation on the structural gene for the neutral protease and a recE4 mutation and growing the resulting clones,

(d) isolating the resulting clones of step (c) containing the hybrid recombinant plasmid,

(e) culturing the resulting clones of step (d) in a liquid culture medium under conditions which result in the production of the neutral protease, and

(f) isolating the neutral protease so produced from the culture medium.

According to the present invention, the hybrid recombinant plasmid is prepared by known methods with the use of DNA from the donor microorganism, which contains the gene which codes for the neutral protease.

In accordance with the present invention, the construction of the hybrid recombinant plasmids comprises:

1. isolating and purifying the chromosomal DNA from the donor microorganism,
2. partially cutting the DNA with a restriction enzyme,
3. isolating the fragments of chromosomal DNA having dimensions between 1.5 and 4 x 10³ base pairs,
4. cutting a plasmid with a restriction enzyme which generates cohesive ends the same as those of the DNA fragments,
5. ligase reaction of the fragments obtained in 3) and the linear DNA of the plasmid.

Among the donor microorganisms suitable for the purpose, the selected strain is B. subtilis BGSC 1A341 (obtained from the Bacillus Genetic Stock Center, Ohio, U.S.A.) which contains the gene which codes for the neutral protease in its chromosome.

The chromosomal DNA of this strain is isolated and purified by one of the known methods.

The DNA obtained is then partially digested with a specific restriction enzyme.

The restriction enzyme MboI is particularly suitable.

The partial digestion of the chromosomal DNA generates fragments of different sizes. From these, fragments having dimensions of between 1.5 and 4 x 10³ base pairs which contain the gene under consideration are isolated by means of a sucrose gradient. Any vector which is compatible with the host microorganism may be used to construct the hybrid recombinant plasmids of the present invention.

These plasmids are easily identifiable if the vector has a marker. Vectors which contain a marker comprising resistance to an antibiotic are particularly suitable. Of these vectors, those preferred are plasmids originally isolated from Staphilococcus aureus and, of these, the plasmid pUB-110 (BGSC 1E6) which contains the gene which gives resistance to Kanamycin.

The said plasmid is cut by known methods by the restriction enzyme BamHI, which generates cohesive ends the same as those generated by the enzyme MboI and which can thus be linked together. In accordance with the present invention, the stage 5) ligase reaction between the DNA fragments of predetermined dimensions and the linearised plasmid pUB-110 is carried out by known methods in the presence of a ligase enzyme. A ligase which is suitable for this purpose is T4 DNA ligase which is commercially available. The reaction being carried out under the conditions indicated above, hybrid recombinant plasmids are obtained which are subsequently used to transform host cells of a suitable microorganism.

In accordance with the present invention it has been found that the host cells which are particularly suitable for this purpose are those obtained by mutation of the strain B. subtilis BGSC 1A341.

The mutant obtained by us, and indicated SMS 108, has two basic characteristics:

1) presence of a mutation in the structural gene for the neutral protease located in the cell genome.
2) presence of a recE4 mutation which prevents homologous recombination in B. subtilis.

The presence of a mutation in the structural gene for the neutral protease results in a neutral protease minus genotype (npr⁻) which facilitates the identification of the strains, when they are transformed by recombinant hybrid plasmids containing the gene which codes the neutral protease.

In the cloning of a chromosomal gene of a microorganism, one of the techniques used is that known as the "shot gun technique". This technique consists in the cutting of the chromosomal DNA with a restriction enzyme and the linking of the fragments obtained, by means of the enzyme T4 DNA ligase, to a vector plasmid in its turn cut by a suitable restriction enzyme. When carried out in this manner, it is possible to obtain a very large series of hybrid molecules constituted by the plasmid DNA and one or more fragments of the chromosomal DNA.

The said molecules are isolated by insertion in host cells. The insertion may be achieved by bringing the hybrid plasmids into contact with microbe cells, the said host cells, by one of the known methods. The plasmid DNA passes through the cell wall and membrane into the cytoplasm where it can become functional. In such a case the cell is said to be transformed. The plasmid transformation has a low efficiency and, on average, only one recombinant hybrid plasmid becomes functional in a single cell.

This permits the obtaining of a population of cells which carry single recombinant hybrid plasmids and which are able to generate colonies on a solid medium. The identification of the colony containing the gene of interest is the simpler, the more efficient and rapid the selection test.

In particular, in order to facilitate the identification of the transformed colonies which contain a hybrid recombinant plasmid containing the functional gene for the neutral protease, it is thought to be advantageous to use a mutant strain of B. subtilis 1A341, which is no longer able to produce the neutral protease

3

because of a mutation in its structural gene. The colonies of this mutant strain, grown on a solid medium containing casein in addition to the substances necessary for bacterial growth, do not generate the typical halo which is visible, on the other hand, around colonies of the parental strain BGSC 1A341.

The lack of caseinolytic activity in the mutant strain is linked to the functional lack of the gene which codes for the neutral protease.

In accordance with the present invention, when these mutant cells are used in the transformation processes, the presence in them of the hybrid recombinant plasmid containing the gene which codes for the neutral protease is made evident on the solid medium, containing casein, by the appearance of a halo around the colony.

The mutation of the parental strain B. subtilis BGSC 1A341 may be achieved by treatment with any one of the mutagenic agents known in the art.

In particular N-methyl-N'-nitro-N-nitrosoguanidine may be used.

The treatment with the said compound is carried out in a buffer solution (pH 6.0), at a temperature of 37°C for a period of about 30 minutes. The gene mutation responsible for the neutral protease minus phenotype is then transferred by transformation into cells of B. subtilis SMS 003, characterised by the markers his, leu, met, thus obtaining a mutant strain his leu, met, npr⁻. The strain B. subtilis SMS 003 has been deposited at the Federal Research Collection, North Central Region, Northern Regional Research Center, (Peoria - Illinois) on September 4, 1984 under the number NRRL - B - 15897.

In accordance with the present invention, the mutant strain npr⁻ is further modified by the introduction of a recE4 mutation which prevents homologous recombination in B. subtilis. When exogenous DNA is introduced into cells of B. subtilis, if it has regions homologous with the chromosomal DNA, it is subject to recombination in the presence of the product of the recE gene.

This recombination causes the insertion of the exogenous DNA fragment into the cell genome.

If the homologous DNA fragment is inserted in a plasmid used to transform cells of B. subtilis recE$^+$, the plasmid may loose the said fragment by recombination.

If, however, the function of the rec-E gene is destroyed in a host cell, the homologous DNA may be maintained on the molecule of the hybrid plasmid and thus remain separated from the chromosomal DNA. In accordance with this and according to the present invention, it has been found extremely advantageous to introduce a recE4 mutation which prevents homologous recombination in the mutant strain SMS003 npr⁻.

The recE4 mutation is isolated by known methods from the chromosomal DNA of the strain B. subtilis BGSC 1A46 and is subsequently introduced into the mutant strain SMS003 by transformation.

The strain obtained, characterised by his, leu, met, recE4, npr⁻ markers and indicated SMS108 by us has been deposited at the Federal Research Collection, North Central Region, Northern Regional Research Center, (Peoria - Illinois) on September 4, 1984 under the number NRRL - B - 15898.

According to the present invention, in stage c), the hybrid recombinant plasmids obtained as described above are introduced into cells of B. subtilis SMS108, rendered competent by the method described by S. Contente and D. Dubnau in Mol.Gen.Genet. 167, (1979) 251-258.

The clones carrying the gene for the neutral protease are selected on plates containing a suitable culture medium to which kanamycin and casein have been added.

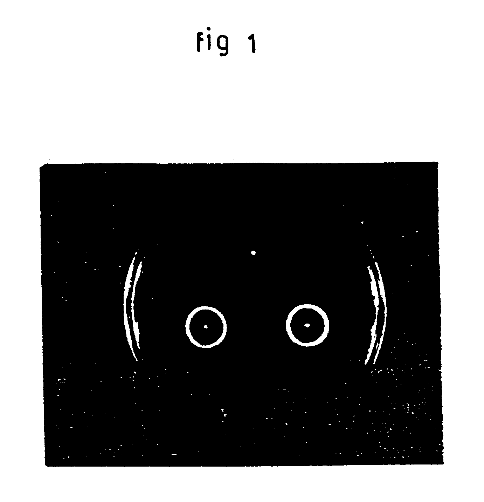

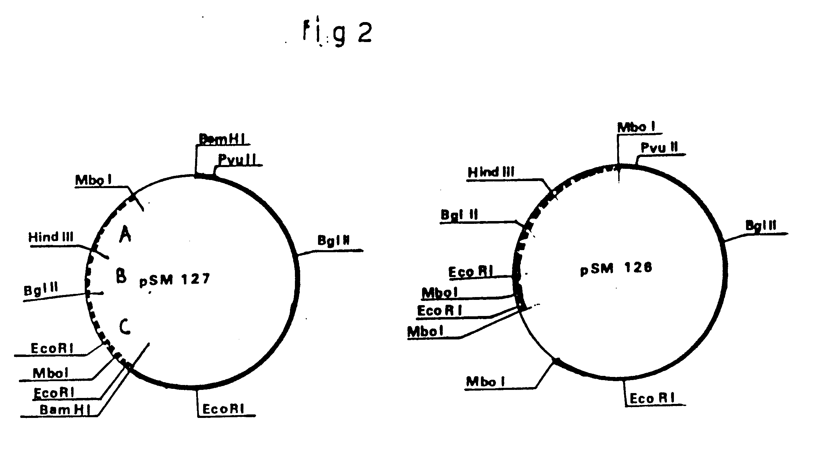

By this method one can obtain only those cells which contain the hybrid recombinant plasmid with the determinant for resistance to kanamycin and, from these, one can easily isolate those containing the gene for the neutral protease, identifiable by the presence of a halo (Figure 1). The hybrid recombinant plasmids which contain the gene for the neutral protease are then isolated from two clones surrounded by haloes. The said plasmids, indicated pSM 126 and pSM 127 give the restriction maps given in Figure 2.

The cells from which the said plasmids, indicated B. subtilis SMS 108 (pSM 126) and B. subtilis SMS 108 (pSM 127), were isolated have been deposited at the Federal Research Collection, North Central Region, Northern Regional Research Center (Peoria - Illinois). on September 4, 1984 under the number NRRL - B - 15899 and NRRL - B - 15900, respectively.

Cells of B. subtilis SMS 108 (pSM 126) and B. subtilis SMS 108 (pSM 127) are then grown under aerobic conditions in a liquid culture medium in the presence of carbon sources, nitrogen sources and mineral salt, at a temperature of 37°C until the neutral protease is obtained in high yields in the culture. medium. Suitable carbon sources which may be used in the culture medium may be of any type of assimilable molecule.

The nitrogen compounds are generally chosen from organic and inorganic ammonium salts such as: ammonium sulphate, ammonium chloride, ammonium nitrate, ammonium acetate, ammonium succinate and urea. Suitable inorganic salts for the purpose include potassium phosphate, sodium phosphate, magnesium sulphate, calcium carbonate, ferrous sulphate, manganese sulphite, zinc sulphate.

These salts are used in quantities commonly used in the usual fermentation processes. According to the present invention the medium used is preferably VY (veal infusion broth (DIFCO), to which yeast extract (DIFCO) has been added.

At the end of the fermentation reaction, the cells are removed from the reaction environment by filtration or centrifugation and the neutral protease produced is determined in the supernatant liquid by measurement of the enzyme activity, according to the method of H. Uheara et al (J. Bacteriol, 119, 82-91, 1971).

The values obtained for the strain B. subtilis SMS108 (pSM126) and B. subtilis SMS108 (pSM127), expressed as units/ml, are 1895 and 2097 respectively.

Analysis of an aliquot of the supernatant liquid on a gel of sodium dodecyl sulphate (SDS) - polyacrylamide by the method of Laemnli U.K. (Nature 277, 680 (1970)) shows, for both strains, the presence of a protein having a molecular weight MW 39,000 identical to that of the neutral protease produced by B. subtilis

In accordance with the present invention the nucleotide sequence of the gene which codes for the neutral protease was determined.

The analysis was carried out by the insertion of restriction fragments A, B and C (Figure 2) of the gene for the neutral protease present in the plasmid pSM127 in the vectors M13mp8 and M13mp9 (obtained from New England, Nuclear) and sequenced by the method of F. Sanger et al (PNAS 74, 5463-5467, 1977) and according to the strategy of M. Poncz et al (PNAS 79, 4298-4302, 1982).

P. Mantsala and H. Zalkin, in J. Bacteriol, 14. 493-501 (1980), reported the terminal amino sequence of the first 16 aminoacids of the extracellular neutral protease of B. subtilis; Ala-Ala-Ala-Thr-Gly-Ser-Gly-Thr -Thr-Leu-Lys-Gly-Ala-Thr-Val-Pro. From a comparison of this data from the literature with the nucleotide sequence which we have determined and which is given in Figure 3, it may be seen that the sequence of 16 aminoacids in the neutral protease is present in the protein derived from the DNA, starting from the aminoacid residue 222. This implies that the gene which we have cloned and which is expressed with a high efficiency in B. subtilis SMS108 is that for the neutral protease: This enzyme is synthesised by B. subtilis SMS108 in the form of a precursor and subsequently is cut at the 221-222 position to give the mature protease.

Description of the drawings

Figure 1 shows a plate containing casein (DIFCO) on which colonies which produce neutral protease are seen surrounded by halos and those which do not produce the enzyme are free from halos.

Figure 2 shows the restriction maps of the hybrid recombinant plasmids pSM126 and pSM127.

Figure 3 is the nucleotide sequence of the gene which codes for the neutral protease. The broken line indicates the sequence of the 16 amino terminal aminoacids of the extra-cellular neutral protease.

The examples below are illustrative and non-limiting of the invention.

Example 1

Preparation of the mutant strain B. subtilis SMS108

Cells of Bacillus subtilis BGSC 1A341 which produce neutral protease are cultivated for one night at 37°C with vigorous agitation in 100 ml of VY culture medium previously sterilised at 120° for 15 minutes and having the following composition:

| | |
|---|---|
| DIFCO Veal Infusion broth | 25 g/l |
| DIFCO Yeast Extract | 5 g/l |
| $H_2O$ | 1 l |

The culture obtained is diluted 1:100 with VY culture medium and grown under vigorous agitation at 37°C.

The growth is followed by measurement of the optical density (O.D.) with a spectro-photometer (Perkin-Elmer model 551 S) at 650 nm in a 1 ml cell with a 1 cm optical path. At on O.D. 650 value of 0.7, or about 0.7, 10 ml of the culture are centrifuged for 20 minutes at 5,000 rpm in a Beckman model 4226 centrifuge. The cells are then washed with 5 ml of Trio maleate (TM) buffer having the following composition per litre:

| Tris | 0.05 M |
|---|---|
| maleic acid | 0.05 M |
| $(NH_4)_2 SO_4$ | 1 g |
| $Mg SO_4 . 7H_4O$ | 0.1 g |
| $Ca (NO_3)_2$ | 0.25 mg |
| $Fe SO_4 . 7H_2O$ | 0.25 mg |
| pH 6.0 | ; |

and then resuspended in 10 ml of TM buffer containing 300 $\mu$g/ml of N-methyl-N'-nitro-N'nitrosoguanidine.

The suspension is kept under agitation at 37°C for 30 minutes.

At the end of the reaction, the suspension is centrifuged and the cells washed with 5 ml of TM buffer and resuspended in 50 ml of VY culture medium.

The suspension is then divided into 50 aliquots of 1 ml each, and each aliquot is grown for one night at 37°C under agitation. 200 $\mu$l of sterile glycerol are then added to each aliquot and after freezing in a dry-ice - ethanol bath, they are kept at -80°C.

In order to isolate the mutants, the various aliquots are brought to a temperature of 37° and then diluted suitably with Spizizen mineral medium and 0.1 ml of each dilution is plated on plates of medium having the following composition per litre:

| DIFCO nutrient broth | 8 g |
|---|---|
| $Mg SO_4 . 7H_2O$ | 0.25 g |
| K Cl | 1 g |
| DIFCO Agar | 15 g |
| $Fe SO_4 . 7H_2O$ | 0.28 g |
| $Mn Cl_2$ | 1.25 mg |
| $Ca (NO_3)_2$ | 164 mg |
| Casein | 10 g |
| $H_2O$ | 1 l |

The plates are incubated at 48°C for one night and then the colonies which are not surrounded by a halo are selected. Of about 13,000 colonies selected, only 10 are found to be negative neutral protease mutants.

One of these mutants was termed SMS104.

The gene mutation responsible for the neutral protease phenotype is then transferred, by transformations into competent cells of the strain B. subtilis SMS003 isolated in our laboratory.

The chromosomal DNA of the strain SMS104, isolated by known methods and as described in example 2, is added to cells of the strain SMS003 rendered competent at a concentration of 1 $\mu$g/ml. Suitable dilutions of the transformation mixture are then plated on casein plates. After a period of incubation of one night at 37°C, one colony of the 500 obtained is not surrounded by a halo of caseinolytic activity. The mutation RecE4 is introduced into this clone, termed SMS107. The chromosomal DNA of B.subtilis BGSC 1A46 (TrpC2, recE4, Thr-5) is extracted and purified by known methods and used, at a concentration of 1 $\mu$g/ml to transform competent cells of SMS107.

The transformation mixture is then plated on minimum medium (Spizizen minimal medium) supplemented with 50 $\mu$g/ml of histidine and leucine. Thus the cells of SMS107 capable of growing on a medium without methionine are selected.

The Met[+] colonies are then transferred to plates of Spizizen medium containing 100 $\mu$g/ml of methylmethane sulphonate. The colonies sensitive to this product are those which have the mutation RecE4. One of these colonies is isolated and termed SMS108.

Example 2

Isolation and purification of the chromosomal DNA of the strain B. subtilis BGSC 1A341

One litre of VY liquid medium in a 3 litre Erlenmeyer flask is innoculated with 10 ml of a 16-hour culture of B. subtilis BGSC 1A341.

The suspension is kept at a temperature of 37°C under vigorous agitation.

The growth is followed by measurement of the optical density (O.D.) with a Perkin Elmer, model 551S spectrophotometer at 650 nm in a 1 ml cell with a 1 cm optical path. At an $OD_{650}$ value of 1, the culture is centrifuged for 20 minutes at 5,000 rpm in a Sorvall centrifuge with a model G53 rotor. The cells are then resuspended in 10 ml of a solution of 0.1 M ethylenediamine tetracetic acid (EDTA) and 0.05 M NaCl, pH 6.9. 1 ml of a solution containing 0.1 M EDTA, 0.05 M NaCl, pH 6.9 and 10 mg/ml of lysozyme (obtained from Boehringer Manheim) are subsequently added to the suspension.

The suspension is kept at 37°C for 30 minutes. At the end of this period, 1 ml of a 10% solution of sodium dodecyl sulphate is added and the suspension is kept at a temperature of 65°C for 10 minutes. To the solution obtained there is then added pronase (obtained from Boehringer Manheim) previously incubated at 37°C for 30 minutes in a solution (SSC) containing 0.15 M NaCl and 0.015 M sodium citrate, up to a final concentration of 1 mg/ml.

The solution is kept at 37°C until it has clarified completely and then NaCl is added up to a final concentration of 1 M.

The precipitated DNA is collected on a glass rod and suspended in a 100 ml beaker containing 3 volumes of cold ethanol. The DNA is subsequently resuspended in 10 ml of SSC 0.1 X and the suspension is kept under mild agitation at room temperature (20° to 25°C) for one night.

At the end of this period, pancreatic RNAse (10 μg/ml) and RNAse T1 (5 μunits/ml) are added and the mixture is incubated at 37°C for 30 minutes.

The proteins present in the mixture are removed by two successive extractions with equal volumes of phenol saturated with SSC and the solution containing the DNA is dialysed against SSC buffer.

The high molecular weight DNA (chromosomal DNA) is then purified by the addition of a 1/10 volume of 3 M $CH_3COONH_4$, 1mM EDTA (pH 7.5) and 0.54 volumes of isopropanol. The DNA thus obtained is recovered and resuspended in SSC buffer, to a final concentration of 1 mg/ml, and kept at 4°C.

Example 3

Partial digestion of the chromosomal DNA with MboI and its fractionation

100 μg of chromosomal DNA obtained as described in Example 2 are suspended in 1 ml of a 6mM solution of Tris-HCl pH7.4, 100 mM NaCl and 6 mM $MgCl_2$ in the presence of 100 units of the enzyme MboI (BRL).

The reaction is carried out at a temperature of 37°C for 30 minutes and then blocked, the temperature being kept at 65°C for 10 minutes.

The solution obtained, containing the partially digested DNA, is divided into three aliquots and then stratified on preformed 10-40% sucrose gradients in the presence of 30 mN Tris -(hydroxymethyl) aminomethane hydrochloride (Tris-HCl), (pH 8.1), 10 mM EDTA, 1M NACl and centrifuged for 20 hours at 25.000 rpm in a Beckmann model SW 27 centrifuge.

2 ml fractions containing fragments of chromosomal DNA of different dimensions are collected. An aliquot of each fraction is analysed on agarose gel with the use of suitable molecular-weight standards to determine the dimensions of the DNA fragments present in the individual fractions.

The fractions containing DNA fragments having dimensions of between 1.5 and $4 \times 10^3$ base pairs (bp) are combined and diluted with an equal volume of water. To this solution are added two volumes of ethanol. The solution is kept in a dry ice-ethanol bath (T = -80°C) for 15 minutes. The precipitated DNA is then separated from the mixture by centrifugation at 10,000 rpm.

The DNA obtained is washed with 70% ethanol and dried under vacuum.

Example 4

Construction of hybrid plasmids containing the gene for the neutral protease

5 μg of the plasmid pUB110 (BGSC 1E6) containing the determinant for resistance to kanamycin are linearised in 50 μl of a solution containing 6 mM Tris-HCl, pH7.4, 100 mM NaCl and 6 mM $MgCl_2$, with 5 units (U) of the restriction enzyme Bam HI (provided by BRL) at a temperature of 37°C for one hour.

5 μg of chromosomal DNA fragments with dimension of between 1.5 and $4 \times 10^3$ b.p., obtained as described in Example 3, and 5 μg of pUB110 linearised as described above, are mixed in 100 μl of a solution containing 20 mM Tris-HCl (pH 7.6), 10 mM $MgCl_2$, 10 mM dithiothreitol and 0.6 mM adenosine triphosphate (ATP) and linked with the use of 10 U of DNA ligase T4. The ligation mixture is kept at room temperature (20° to 25°C) for 3 hours.

Example 5

Transformation of cells of B. subtilis SMS108

Cells of B. subtilis SMS108 are transformed by the method of D. Dubnau et al. (J. Mol. Biol. 56, 209-221, 1971).

20 $\mu$l of the ligation mixture obtained as explained in Example 4 are used to transform 1 ml of B. subtilis SMS108 cells which have been rendered competent.

The transformation mixture is kept at 37°C for 30 minutes and subsequently spread on plates containing a casein medium the composition of which is given in Example 1.

The plates are incubated at 37°C for 16 hours. At the end of this period colonies appear several of which are surrounded by halos. These are the colonies of B. subtilis SMS108 containing the hybrid plasmid with the determinant for resistance to kanamycin and the gene for the extracellular neutral protease which causes enzymatic hydrolysis of the casein present. Of 130,000 transformants, 12 appear surrounded by a halo of caseinolytic activity.

Example 6

Isolation and characterisation of the hybrid plasmids containing the gene which codes for the neutral protease

From two transformants having caseinolytic activity obtained as in Example 5, the hybrid plasmids pSM126 and pSM127, whose restriction maps are given in Figure 2, are isolated by the method described by Gryczan et al (J. Bacteriology 134 318, 329 - 1978).

These plasmids have a common zone of DNA of about 2400 base pairs (bp). The plasmids thus isolated are reintroduced separately into competent cells of the strain B. subtilis SMS108.

The transformation mixtures, after growth at 37°C for 30 minutes, are spread on plates containing a casein medium having the composition described previously. After a period of 16 hours at a temperature of 37°C, all the transformants resistant to kanamycin appear surrounded by a halo of proteolytic activity.

This demonstrates presence on the two hybrid plasmids pSM126 and pSM127 of the gene which codes for an extracellular protease.

In the same experiment, cells of B. subtilis SMS108 are transformed by the plasmid pUB110, containing the determinant for resistance to kanamycin.

The transformed cells, spread on plates containing casein in the medium do not, after 16 hours at 37°C, give rise to any halo formation. This shows that these transformants are free from genes codifying for caseinolytic activity.

Example 7

Production and characterisation of the proteolytic activity of the strain B. subtilis SMS108 (pSM126) and B. subtilis SMS108 (pSM127)

Four 100 ml Erlenmeyer flasks containing 10 ml of VY medium, to the first 3 of which 5 $\mu$g/ml of kanamycin have been added, are innoculated respectively with the following strains:B. subtilis SMS108 (pSM126), B. subtilis SMS108 (pSM127), SMS108 (pUB110) and B. subtilis BGSC 1A341. The flasks are kept at a temperature of 37°C for 24 hours under vigorous agitation.

At the end of this period, the cells are removed from the culture media by centrifuging and the supernatant liquids are dialysed at 4°C for 24 hours against 10 mM Tris-HCl (pH 7.5) buffer containing 2 mM calcium acetate.

100 $\mu$l of each supernatant liquid are then suitably diluted with 10 mH Tris-HCl (pH 7.5) buffer containing 2mM calcium acetate and mixed with 0.5 ml of a 0.6% casein solution (Merck) in 50 mM Tris-HCl buffer, in order to determine the activity of the enzyme produced by each strain in the culture broth.

The mixtures are incubated at a temperature of 37°C for 30 minutes. The non-hydrolysed casein is then precipitated by the addition of 0.5 ml of a solution containing 0.11M trichloracetic acid, 0.33M acetic acid and 0.22M sodium acetate at room temperature (20° to 25°C) for 30 minutes.

At the end of this period, the preceipitate is separated by centrifugation at 10,000 rpm for 10 minutes and the absorbance of the supernatant liquid is measured at a wavelength of 275nm, with the use of a Perkin Elmer model 551S spectrophotometer.

A protease unit is defined as the quantity of enzyme which, in one minute at 37°C, under the conditions described above, causes an increase in absorbance at 275 nm equivalent to 1 $\mu$g of tyrosine.

The characterisation of the protease produced by the individual strains was effected by determining separately the activity of the neutral protease and of the serine protease present in the same solution.

The first is inhibited by pre-incubation of the supernatant liquid for one hour at 0°C in the presence of 5 mM EDTA; the second is inhibited by maintaining the supernatant liquid at 0°C for one hour in the presence of 1 mM phenylmethylsulphonyl fluoride (PSMF).

The results obtained from the analysis of the supernatant liquids for the various strains are given in Table 1.

TABLE 1

| Proteolitic activity in the culture medium (unit/ml) | | | |
|---|---|---|---|
| Strain | Absence of inhibitors | in the presence of EDTA | in the presence of PMFS |
| 1) B.subtilis SMS108 (pSM126) | 1,939 | *N.D. | 1,895 |
| 2) B.subtilis SMS108 (pSM127) | 2,190 | *N.D. | 2,097 |
| 3) B.subtilis SMS108 (pUB110) | 2.9 | 3.1 | 0.5 |
| 4) B.subtilis BGSC 1A341 | 346 | *N.D. | 328 |

*N.D. (not determinable).

In the presence of high quantities of neutral protease it is not possible to determine the activity due to the serine protease.

From the data given in Table I it is seen that the strains of the present invention produce a neutral protease with a yield 5-6 times greater than the original strain.

**Claims**

1. A method for the production of Bacillus subtilis neutral protease comprising:
    (a) isolating, from the chromosomal DNA of Bacillus subtilis BGSG 1A341, the gene coding the neutral protease having the sequence recited in Figure 3,
    (b) constructing a hybrid recombinant plasmid containing the gene obtained in step (a),
    (c) introducing the hybrid recombinant plasmid of step (b) into Bacillus subtilis SMS 108 (NRRL-B-1589) having a mutation on the structural gene for the neutral protease and a recE4 mutation and growing the resulting clones,
    (d) isolating the resulting clones of step (c) containing the hybrid recombinant plasmid,
    (e) culturing the resulting clones of step (d) in a liquid culture medium under conditions which result in the production of the neutral protease, and
    (f) isolating the neutral protease so produced from the culture medium.

2. The method according to Claim 1, wherein the mutations are obtained by:
    (a') treating Bacillus strain BGSG 1A341, which can produce the neutral protease, with a mutagenic agent,
    (b') transferring the gene mutation achieved to a strain of Bacillus subtilis, and
    (c') introducing a recE4 mutation into the transformed Bacillus subtilis strain obtained in step (b').

3. The method according to Claim 2, wherein the mutagenic agent in step (a') is N-methyl-N'-nitro-N-nitrosoguanidine.

4. The method according to Claim 2, wherein the Bacillus subtilis strain in step (b') is Bacillus subtilis SMS 003 (NRRL-B-15897).

5. The method according to Claim 2, wherein the introduction of the recE4 mutation in step (c') is effected by transformation with a gene containing a recE4 mutation.

6. The method according to claim 5, wherein the gene containing the recE4 mutation is isolated from the chromosomal DNA of Bacillus subtilis BGSC 1A46.

**7.** The method according to any of Claims 1 to 6, wherein the introduction of the hybrid recombinant plasmid in step (c) is carried out by transformation.

**8.** The method according to any of Claims 1 to 7 wherein the isolation of clones containing the recombinant hybrid plasmid in step (d) is carried out by plating the clones of step (c) on a culture medium to which kanamycin and casein have been added.

**9.** The method according to any of Claims 1 to 8 wherein the growth in step (c) is carried out in a liquid medium under aerobic conditions, in the presence of nitrogen sources, carbon sources, mineral salts, and at a temperature of from 20° to 40°C.

**10.** The method according to any of Claims 1 to 9 wherein the clone isolated in step (d) is Bacillus subtilis SMS 108 (pSM 126) (NRRL-B-15899) or Bacillus subtilis SMS 108 (pSM 127) (NRRL-B-15900).

**11.** The method according to any of Claims 1 to 10 wherein the recombinant hybrid plasmid of step (b) is prepared by:
(a") isolating and purifying chromosomal DNA from Bacillus subtilis BGSC 1A341,
(b") partially cutting the chromosomal DNA with the restriction enzyme MboI,
(c") isolating fragments of the chromosomal DNA of between 1.5 and $4 \times 10^3$ base pairs in length,
(d") cutting a plasmid with a restriction enzyme, and
(e") ligating, in the presence of T4 DNA ligase, the DNA fragments obtained in step (c") with the cut plasmid obtained in step (d") so as to obtain said recombinant hybrid plasmid.

**12.** The method according to Claim 11, wherein the isolation of fragments in step (c") is carried out on a sucrose gradient.

**13.** The method according to Claim 11, wherein the plasmid of step (d") is pUB110 (BGSG 1E6) and the restriction enzyme is BamHI.

**14.** The method according to Claim 1, wherein the hybrid plasmid of step (b) is pSM 126 or pSM 127 as defined in Claims 15 and 16, respectively.

**15.** Plasmid pSM 126 as obtainable from Bacillus subtilis strain SMS 108 (pSM 126) deposited with NRRL under accession number NRRL-B-15899.

**16.** Plasmid pSM 127 as obtainable from Bacillus subtilis SMS 108 (pSM 127) deposited with NRRL under accession number NRRL-B-15900.

**17.** A method for the production of Bacillus subtilis neutral protease comprising the step of culturing a Bacillus subtilis strain selected from the group consisting of Bacillus subtilis SMS 108 (pSM 126) (NRRL-B-15899) or Bacillus subtilis SMS 108 (pSM 127) (NRRL-B-15900) in a liquid culture medium under conditions which result in the production of neutral protease.

**Revendications**

**1.** Procédé pour la production de protéase neutre de Bacillus subtilis comprenant :
(a) l'isolement, à partir de l'ADN chromosomique de Bacillus subtilis BGSG 1A341, du gène codant pour la protéase neutre ayant la séquence illustrée par la figure 3,
(b) la construction d'un plasmide recombinant hybride contenant le gène obtenu dans l'étape (a),
(c) l'introduction du plasmide recombinant hybride de l'étape (b) dans Bacillus subtilis SMS 108 (NRRL-B-1589) ayant une mutation du gène structural pour la protéase neutre et une mutation recE4 et la culture des clones obtenus,
(d) l'isolement des clones obtenus de l'étape (c) contenant le plasmide recombinant hybride,
(e) la culture des clones obtenus de l'étape (d) dans un milieu de culture liquide dans des conditions qui conduisent à la production de la protéase neutre, et
(f) l'isolement de la protéase neutre ainsi produite à partir du milieu de culture.

**2.** Procédé selon la revendication 1, dans lequel les mutations sont obtenues par :

(a') traitement de la souche de Bacillus BGSG 1A341, qui peut produire la protéase neutre, avec un agent mutagène,

(b') transfert de la mutation génétique obtenue à une souche de Bacillus subtilis et

(c') introduction d'une mutation recE4 dans la souche de Bacillus subtilis transformée obtenue dans l'étape (b').

3. Procédé selon la revendication 2, dans lequel l'agent mutagène, dans l'étape (a'), est la N-méthyl-N'-nitro-N-nitrosoguanidine.

4. Procédé selon la revendication 2, dans lequel la souche de Bacillus subtilis, dans l'étape (b'), est Bacillus subtilis SMS 003 (NRRL-B-15897).

5. Procédé selon la revendication 2, dans lequel l'introduction de la mutation recE4, dans l'étape (c'), est effectuée par transformation avec un gène contenant une mutation recE4.

6. Procédé selon la revendication 5, dans lequel le gène contenant la mutation recE4 est isolé de l'ADN chromosomique de Bacillus subtilis BGSC 1A46.

7. Procédé selon l'une quelconque des revendications 1 à 6, dans lequel l'introduction du plasmide recombinant hybride, dans l'étape (c), est effectuée par transformation.

8. Procédé selon l'une quelconque des revendications 1 à 7, dans lequel l'isolement des clones contenant le plasmide hybride recombinant de l'étape (d) est effectué par étalement des clones de l'étape (c) sur un milieu de culture auquel on a ajouté de la kanamycine et de la caséine.

9. Procédé selon l'une quelconque des revendications 1 à 8, dans lequel la culture, dans l'étape (c), est effectuée dans un milieu liquide en conditions aérobies, en présence de sources d'azote, de sources de carbone, de sels minéraux et à une température de 20 à 40°C.

10. Procédé selon l'une quelconque des revendications 1 à 9, dans lequel le clone isolé dans l'étape (d) est Bacillus subtilis SMS 108 (pSM 126) (NRRL-B-15899) ou Bacillus subtilis SMS 108 (pSM 127) (NRRL-B-15900).

11. Procédé selon l'une quelconque des revendications 1 à 10, dans lequel le plasmide hybride recombinant de l'étape (b) est préparé par :

(a'') isolement et purification d'ADN chromosomique de Bacillus subtilis BGSC 1A341,

(b'') coupure partielle de l'ADN chromosomique avec l'enzyme de restriction Mbo I,

(c'') isolement de fragments de l'ADN chromosomique ayant une longueur entre 1,5 et 4 x $10^3$ paires de bases,

(d'') coupure d'un plasmide avec une enzyme de restriction et

(e'') ligature, en présence d'ADN ligase de T4, des fragments d'ADN obtenus dans l'étape (c'') avec le plasmide coupé obtenu dans l'étape (d'') de façon à obtenir ledit plasmide hybride recombinant.

12. Procédé selon la revendication 11, dans lequel l'isolement des fragments de l'étape (c'') est effectué sur un gradient de saccharose.

13. Procédé selon la revendication 11, dans lequel le plasmide de l'étape (d'') est pUB110 (BGSG 1E6) et l'enzyme de restriction est BamHI.

14. Procédé selon la revendication 1, dans lequel le plasmide hybride de l'étape (b) est pSM 126 ou pSM 127, comme définis respectivement dans les revendications 15 et 16.

15. Plasmide pSM 126 pouvant être obtenu à partir de la souche de Bacillus subtilis SMS 108 (pSM 126) déposée au NRRL sous le numéro d'enregistrement NRRL-B-15899.

16. Plasmide pSM 127 pouvant être obtenu à partir de Bacillus subtilis SMS 108 (pSM 127) déposée au NRRL sous le numéro d'enregistrement NRRL-B-15900.

**17.** Procédé pour la production de la protéase neutre de Bacillus subtilis comprenant l'étape de culture d'une souche de Bacillus subtilis choisie dans le groupe constitué par Bacillus subtilis SMS 108 (pSM 126) (NRRL-B-15899) et Bacillus subtilis SMS 108 (pSM 127) (NRRL-B-15900), dans un milieu de culture liquide, dans des conditions qui assurent la production de protéase neutre.

**Patentansprüche**

**1.** Verfahren zur Herstellung neutraler Bacillus subtilis Protease, umfassend:
(a) die Isolierung des die neutrale Protease codierenden Gens mit der in Abbildung 3 angeführten Sequenz aus der chromosomalen DNA von Bacillus subtilis BGSG 1A341,
(b) der Konstruktion eines hybriden rekombinanten Plasmids, das das in Schritt (a) erhaltene Gen enthält,
(c) das Einführen des hybriden rekombinanten Plasmids aus Schritt (b) in Bacillus subtilis SMS 108 (NRRL-B-1589), der eine Mutation des strukturellen Gens für die neutrale Protease und eine recE4-Mutation aufweist, und Anzüchten der erhaltenen Klone,
(d) das Isolieren der erhaltenen Klone aus Schritt (c), die das hybride rekombinante Plasmid enthalten,
(e) die Züchtung der erhaltenen Klone aus Schritt (d) in einem flüssigen Nährmedium unter Bedingungen, die die Produktion der neutralen Protease ergeben, und
(f) das Isolieren der so hergestellten neutralen Protease aus dem Nährmedium.

**2.** Verfahren nach Anspruch 1, wobei die Mutationen erzielt werden durch:
(a') Behandlung des Bacillus Stamms BGSG 1A341, der die neutrale Protease produzieren kann, mit einem mutagenen Mittel,
(b') Übertragen der erzielten Genmutation auf einen Stamm von Bacillus subtilis, und
(c') Einbringen einer recE4-Mutation in den in Schritt (b') erhaltenen transformierten Bacillus subtilis Stamm.

**3.** Verfahren nach Anspruch 2, wobei das mutagene Mittel in Schritt (a') N-Methyl-N'-nitro-N-nitrosoguanidin ist.

**4.** Verfahren nach Anspruch 2, wobei der Bacillus subtilis Stamm in Schritt (b') Bacillus subtilis SMS 003 (NRRL-B-15897) ist.

**5.** Verfahren nach Anspruch 2, wobei das Einbringen der recE4-Mutation in Schritt (c') durch Transformierung mit einem Gen erreicht wird, das eine recE4-Mutation enthält.

**6.** Verfahren nach Anspruch 5, wobei das die recE4-Mutation enthaltende Gen aus der chromosomalen DNA von Bacillus subtilis BGSC 1A46 isoliert wird.

**7.** Verfahren nach einem der Ansprüche 1 bis 6, wobei die Einführung des hybriden rekombinanten Plasmids in Schritt (c) mittels Transformierung durchgeführt wird.

**8.** Verfahren nach einem der Ansprüche 1 bis 7, wobei die Isolierung der Klone, die das rekombinante hybride Plasmid enthalten, in Schritt (d) durch Plattieren der Klone aus Schritt (c) auf ein Nährmedium, dem Kanamycin und Casein zugesetzt wurden, durchgeführt wird.

**9.** Verfahren nach einem der Ansprüche 1 bis 8, wobei das Wachstum in Schritt (c) durchgeführt wird in einem flüssigen Medium unter aeroben Bedingungen in Anwesenheit von Stickstoffquellen, Kohlenstoffquellen, Mineralsalzen und bei einer Temperatur von 20° bis 40°C.

**10.** Verfahren nach einem der Ansprüche 1 bis 9, wobei der in Schritt (d) isolierte Klon Bacillus subtilis SMS 108 )pSM 126) (NRRL-B-15899) oder Bacillus subtilis SMS 108 (pSM 127) (NRRL-B-15900) ist.

**11.** Verfahren nach einem der Ansprüche 1 bis 10, wobei das rekombinante hybride Plasmid von Schritt (b) hergestellt wird durch:
(a'') Isolierung und Reinigung chromosomaler DNA von Bacillus subtilis BGSC 1A341,
(b'') teilweisem Schneiden der chromosomalen DNA mit dem Restriktionsenzym MboI,

(c'') Isolierung von Fragmenten der chromosomalen DNA mit einer Länge von 1,5 bis $4.10^3$ Basenpaaren,

(d'') Schneiden eines Plasmids mit dem Restriktionsenzym, und

(e'') Binden, in Anwesenheit von T4 DNA Ligase, der in Schritt (c'') erhaltenen DNA-Fragmente mit dem in Schritt (d'') erhaltenen geschnittenen Plasmid, um das erwähnte rekombinante hybride Plasmid zu erhalten.

12. Verfahren nach Anspruch 11, wobei die Isolierung der Fragmente in Schritt (c'') auf einem Saccharose-Gradienten durchgeführt wird.

13. Verfahren nach Anspruch 11, wobei das Plasmid aus Schritt (d'') pUB110 (BGSG 1E6) ist und das Restriktionsenzym BamHI ist.

14. Verfahren nach Anspruch 1, wobei das hybride Plasmid aus Schritt (b) pSM 126 oder pSM 127, wie in Anspruch 15 bzw. 16 definiert, ist.

15. Plasmid pSM 126, wie erhältlich aus Bacillus subtilis Stamm SMS 108 (pSM 126) hinterlegt bei NRRL unter der Eingangszahl NRRL-B-15899.

16. Plasmid pSM 127, wie erhältlich aus Bacillus subtilis Stamm SMS 108 (pSM 127) hinterlegt bei NRRL unter der Eingangszahl NRRL-B-15900.

17. Verfahren zur Herstellung von neutraler Bacillus subtilis Protease, umfassend den Schritt der Züchtung eines Bacillus subtilis Stammes ausgewählt aus der Gruppe bestehend aus Bacillus subtilis SMS 108 (pSM 126) (NRRL-B-15899) oder Bacillus subtilis SMS 108 (pSM 127) (NRRL-B-15900) in einem flüssigen Nährmedium unter Bedingungen, die die Produktion neutraler Protease ergeben.

fig 1

fig 2

```
AAAAATATCTCATCTTCCCCTTGATAAAAATAAACACAGGACAATACTATCAATTTTGTCTAGTTATGTTAGTTTTTGTTAAGTATTCCAGAATGCTGGTTTA

                            RBS              fMet Gly Leu Gly Lys Lys Leu Ser Val Ala Val Ala Ala Ser
ATATAACAATATAAAGTTTTCACTATTTTCAAAAAGCGCGATTTATT GTG GCT TTA GGT AAG AAA TTG TCT GTT GCT GTC GCT GCT TCG   42
                    20                                                                            40
Phe Met Ser Leu Ser Ile Ser Leu Pro Gly Val Gln Ala Ala Glu Gly His Gln Leu Lys Glu Asn Gln Thr Asn Phe
TTT ATG AGT TTA TCA ATC AGC CTG CCA GGT GTT CAG GCT GCT GAA GGT CAT CAG CTT AAA GAG AAT CAA ACA AAT TTC   120
                                                                          60
Leu Ser Lys Asn Ala Ile Ala Gln Ser Glu Leu Ser Ala Pro Asn Asp Lys Ala Val Lys Gln Phe Leu Lys Lys Asn
CTC TCC AAA AAT GCG ATT GCG CAA TCA GAA CTC TCT GCA CCA AAT GAC AAG GCT GTC AAG CAG TTT TTG AAA AAG AAC   198
                                            80
Ser Asn Ile Phe Lys Gly Asp Pro Ser Lys Arg Leu Lys Leu Val Glu Ser Thr Thr Asp Ala Leu Gly Tyr Lys His
AGC AAC ATT TTT AAA GGT GAC CCT TCC AAA AGG CTG AAG CTT GTT GAA AGC ACG ACT GAT GCC CTT GGA TAC AAG CAC   276
                                100               HindIII
Phe Arg Tyr Ala Pro Val Val Asn Gly Val Pro Ile Lys Asp Ser Gln Val Ile Val His Val Asp Lys Ser Asp Asn
TTT CGA TAT GCG CCT GTC GTT AAC GGA GTG CCA ATT AAA GAT TCG CAA GTA ATC GTT CAC GTC GAT AAA TCC GAT AAT   354
        120                                                                       140
Val Tyr Ala Val Asn Gly Glu Leu His Asn Gln Ser Ala Ala Lys Thr Asp Asn Ser Gln Lys Val Ser Ser Glu Lys
GTC TAT GCG GTC AAT GGT GAA TTA CAC AAT CAA TCT GCT GCA AAA ACA CAT AAC AGC CAA AAA GTC TCT TCT GAA AAA   432
                                      160
Ala Leu Ala Leu Ala Phe Lys Ala Ile Gly Lys Ser Pro Asp Ala Val Ser Asn Gly Ala Ala Lys Asn Ser Asn Lys
GCG CTG GCA CTC GCT TTC AAA GCT ATC GGC AAA TCA CCA GAC GCT GTT TCT AAC GGA GCG GCC AAA AAC AGC AAT AAA   510
                              180
Ala Glu Leu Lys Ala Ile Glu Thr Lys Asp Gly Ser Tyr Arg Leu Ala Tyr Asp Val Thr Ile Arg Tyr Val Glu Pro
GCC GAA TTG AAA GCG ATA GAA ACA AAG GAC GGC AGC TAT CGT CTT GCT TAC GAC GTG ACG ATT CGC TAT GTC GAG CCT   588
                200                                                               220
Glu Pro Ala Asn Trp Glu Val Leu Val Asp Ala Glu Thr Gly Ser Ile Leu Lys Gln Gln Asn Lys Val Glu His Ala
GAA CCT GCA AAC TGG GAA GTC TTA GTT GAC GCC GAA ACA GGC AGC ATT TTA AAA CAG CAA AAT AAA GTA GAA CAT GCC   666

Ala Ala Thr Gly Ser Gly Thr Thr Leu Lys Gly Ala Thr Val Pro Leu Asn Ile Ser Tyr Glu Gly Gly Lys Tyr Val
GCC GCC ACT GGA AGC GGA ACA ACT CTA AAG GGC GCA ACT GTT CCT TTG AAC ATC TCT TAT GAA GGC GGA AAA TAT GTT   744
                                  260
Leu Arg Asp Leu Ser Lys Pro Thr Gly Thr Gln Ile Ile Thr Tyr Asp Leu Gln Asn Arg Gln Ser Arg Leu Pro Gly
CTA AGA GAT CTT TCA AAA CCA ACA GGC ACC CAA ATC ATC ACA TAT GAT TTG CAA AAC AGA CAA AGC CGC CTT CCG GGC   822
        BglII             280                                                             300
Thr Leu Val Ser Ser Thr Thr Lys Thr Phe Thr Ser Ser Ser Gln Arg Ala Ala Val Asp Ala His Tyr Asn Leu Gly
ACG CTT GTC TCA AGC ACA ACG AAA ACA TTT ACA TCT TCA TCA CAG CGG GCA GCC GTT GAC GCA CAC TAT AAC CTC GGT   900
                                          320
Lys Val Tyr Asp Tyr Phe Tyr Ser Asn Phe Lys Arg Asn Ser Tyr Asp Asn Lys Gly Ser Lys Ile Val Ser Ser Val
AAA GTG TAT GAT TAT TTT TAT TCA AAC TTT AAA CGA AAC AGC TAT GAT AAC AAA GGC AGT AAA ATC GTT TCT TCC GTT   978
                                  340
His Tyr Gly Thr Gln Tyr Asn Asn Ala Ala Trp Thr Gly Asp Gln Met Ile Tyr Gly Asp Gly Asp Gly Ser Phe Phe
CAC TAC GGC ACT CAA TAC AAT AAC GCT GCA TGG ACA GGA GAC CAG ATG ATT TAC GGT GAT GGC GAC GGT TCA TTC TTC  1056
                          360
Ser Pro Leu Ser Gly Ser Leu Asp Val Thr Ala His Glu Met Thr His Gly Val Thr Gln Glu Thr Ala Asn Leu Ile
TCT CCG CTT TCC GGC TCA TTA GAT GTG ACA GCG CAT GAA ATG ACA CAT GGC GTC ACC CAA GAA ACA GCC AAC TTG ATT  1134
        380                                                                 400
Tyr Glu Asn Gln Pro Gly Ala Leu Asn Glu Ser Phe Ser Asp Val Phe Gly Tyr Phe Asn Asp Thr Glu Asp Trp Asp
TAT GAA AAT CAG CCA GGT GCA TTA AAC GAG TCT TTC TCT GAC GTA TTC GGG TAT TTT AAC GAT ACA GAA GAC TGG GAC  1212
                                    420
Ile Gly Glu Asp Ile Thr Val Ser Gln Pro Ala Leu Arg Ser Leu Ser Asn Pro Thr Lys Tyr Asn Gln Pro Asp Asn
ATC GGT GAA GAC ATT ACG GTC AGC CAG CCT GCT CTT CGC AGC CTG TCC AAC CCT ACA AAA TAC AAC CAG CCT GAC AAT  1290
                          440
Tyr Ala Asn Tyr Arg Asn Leu Pro Asn Thr Asp Glu Gly Asp Tyr Gly Gly Val His Thr Asn Ser Gly Ile Pro Asn
TAC GCC AAT TAC AGA AAC CTT CCA AAC ACA GAT GAA GGC GAT TAT GGC GGT GTA CAC ACA AAC AGC GGA ATT CCA AAC  1368
        460                                                               EcoRI  480
Lys Ala Ala Tyr Asn Thr Ile Thr Lys Leu Gly Val Ser Lys Ser Gln Gln Ile Tyr Tyr Arg Ala Leu Thr Thr Tyr
AAA GCC GCT TAC AAC ACC ATC ACA AAA CTT GGT GTA TCT AAA TCA CAG CAA ATC TAT TAC CGT GCG TTA ACA ACG TAC  1446
                                            500
Leu Thr Pro Ser Ser Thr Phe Lys Asp Ala Lys Ala Ala Leu Ile Gln Ser Ala Arg Asp Leu Tyr Gly Ser Thr Asp
CTC ACG CCT TCT TCC ACG TTC AAA GAT GCC AAG GCA GCT CTC ATT CAG TCT GCC CGT GAC CTC TAC GGC TCA ACT GAT  1524
                          520
Ala Ala Lys Val Glu Ala Ala Trp Asn Ala Val Gly Leu
GCC GCT AAA GTT GAA GCA GCC TGG AAT GCT GTT GGA TTG TAATATTAGGAAAAGCCTGAGATCCCTCAGGCTTTTTTGTTACATATCTTG  1614


ATTTCTCTCTTCAGCAGAAACAACGAAAAGATGCTGTCATGAGACAGCAACCGATTCTGATTTGCAAAAAGAGGGATGCAGCCGCAAGTGCGCATTTTATAAA  1717


AGCTAATGATTCAGTCCACATAATTGATAGACGAATTCTGCTACAGGTTACGTGGCTATGTGAAGGATCC                                  1787
        EcoRI                                         BamHI
```